# EUROPÄISCHE PATENTSCHRIFT

(11) **EP 0 967 274 B1**
(45) Veröffentlichungstag und Bekanntmachung des Hinweises auf die Patenterteilung: **04.02.2004**
(21) Anmeldenummer: 99250187.4
(22) Anmeldetag: 14.06.1999
(51) Int. Cl.: C12N 15/10

(54) **Verfahren zur Herstellung von hantelförmigen DNA-Molekülen als Expressionskonstrukte**
Method for producing dumbbell- shaped DNA molecules as expression constructs
Procédé de production des molécules d'ADN en forme d'haltère et leur utilisation comme vecteurs d'expression

(30) Priorität: 15.06.1998 DE 19826758
(43) Veröffentlichungstag der Anmeldung: 29.12.1999
(73) Patentinhaber: Mologen Forschungs-, Entwicklungs- und Vertriebs GmbH, 14195 Berlin (DE)
(72) Erfinder: Wittig, Burghardt, 14195 Berlin (DE); Junghans, Claas, 14195 Berlin (DE); Schroff, Matthias, 14195 Berlin (DE)
(74) Vertreter: Omsels, Hermann-Josef

(56) Entgegenhaltungen:
- EP-A- 0 430 270
- WO-A-92/13963
- WO-A-93/13216
- WO-A-98/21322

## Beschreibung

Gentherapie und genetische Vakzinierung sind moderne Methoden der molekularen Medizin, deren Anwendung in der Therapie und Prävention von Erkrankungen erhebliche Konsequenzen für die medizinische Praxis haben wird. Beide Methoden beruhen grundsätzlich auf der Einbringung von Nukleinsäuren in Zellen oder Gewebe des Patienten, sowie der anschließenden Verarbeitung der auf den eingebrachten Nukleinsäuren beinhalteten Information ("Expression"). Allerdings bedürfen auf Expressionssystemen beruhende medizinische Methoden vor ihrer breiten Anwendung noch der Lösung einiger grundsätzlicher Probleme.

Eines dieser Probleme ist der sichere, zell- oder gewebespezifische und effiziente Transfer der Nukleinsäuren ins Zielgewebe; ein anderes betrifft die möglichen Wirkungen von Sequenzen, die herstellungs- oder konstruktionsbedingt als Teil der verabreichten Expressionskonstrukte, aber nicht als Teil der gewünschten zu exprimierenden Information an den Wirkort gelangen. Mögliche Lösungen für diese Probleme wurden in unserer Anmeldung "Designprinzip für die Konstruktion von Expressionskonstrukten für die Gentherapie" (Anmeldung WO 98/21322) skizziert. Zentraler Aspekt der dort erörterten Erfindung ist ein hantelförmiges, linear doppelsträngiges DNA-Molekül als Expressionskonstrukt.

Es sind mehrere Methoden zur Herstellung solcher Konstrukte bekannt. Entweder geht man dabei von der Synthese des das Expressionskonstrukt bildenden Doppelstranges durch Amplifikation per Polymerase-Kettenreaktion aus, wobei die entstehenden doppelsträngigen, nicht kovalent-geschlossenen Moleküle dann durch anschließenden Verdau der Enden mit einer Einzetstrang-Überhänge bildenden Restriktionsendonuklease und anschließender Ligation von auf die entstandenen Überhänge passenden Haamadel-Oligomeren zu hantelförmigen Konstrukten modifiziert werden. Dieses Vorgehen führt zu einem relativ einfach aufzureinigenden Rohprodukt aus hantelförmigen Expressionskonstrukten, dem gewünschten Produkt, sowie Verunreinigungen durch Oligomere aus eingesetztem Primer sowie ligierten Haamadel-Dimeren. Allerdings bringt die Synthese durch PCR neben dem ökonomisch ungünstigen Einsatz der thermostabilen DNA-Polymerasen deren relativ hohe Fehlerrate der Synthese mit sich, was zu für pharmazeutische Produkte intolerablen Unreinheiten im Produkt führt.

Alternativ können die Konstrukte als Teil eines in Bakterien replizierbaren Konstruktes biotechnologisch hergestellt werden, anschließend durch Restriktionsverdau von den unerwünschten DNA-Fragmenten, die zur Vermehrung des Konstruktes in den Bakterien dienten, getrennt werden und nach Aufreinigung analog dem für PCR-Produkte skizzierten Vorgehen in hantelförmige Moleküle umgewandelt werden.

Aus der WO 92/13962 (Hyman) ist die grundsätzliche Reinigung von kovalent geschlossenen DNA-Molekülen durch Abbau mit T4- oder T7-Exonukleasen, eine selektive Hydrolyse verunreinigter DNA durch Restriktionsenzyme, die keine Erkennungssequenzen auf den zu reinigenden DNA-Molekülen aufweisen und die anschließende Abtrennung weiterer Verunreinigungen bekannt Dieses Verfahren ist jedoch auf durch Ligation hergestellte DNA-Moleküle nicht anwendbar.

Das Aufreinigen der verschiedenen so aus dem Restriktionsverdau der Plasmide hervorgehenden Fragmente kann technisch sehr schwierig sein, weil die Mindestgröße eines ein bakterielles Resistenzgen enthaltenden Vektorrückgrates in der gleichen Größenordnung wie die gewöhnlicherweise für gentherapeutische Ansätze verwendeten Expressionskassetten liegt, also etwa bei einer bis fünf Kilobasen. Die Baslslinientrennung durch chromatographische Verfahren von Restriktionsfragmenten in der Größenordnung von einem bis fünf Kilobasenpaaren DNA kann, besonders bei größeren Mengen, erhebliche technische Probleme aufwerfen. Eine Methode zur Darstellung der hantelförmigen Expressionskonstrukte, die diese Problematik vermeiden würde, wäre also von erheblichem praktischen Interesse.

Erfindungsgemäße technische Lösung:
Erfindungsgemäß wird das DNA-Molekül, welches den Hauptteil des herzustellenden (both times !) Expressionskonstruktes bildet, durch fermentative Vermehrung und anschließender Isolierung als Teil eines in Bakterien replizierbaren Plasmids synthetisiert. Besagtes DNA-Molekül wird dann aus dem Plasmid, in dem es in einfacher oder mehrfacher Kopie vorliegen kann, durch Verdau mit Restriktionsendonukleasen ausgeschnitten, die an den entstehenden Schnittstellen kurze einzelsträngige Überhänge bilden. In einem anschließenden Schritt wird das Rohgemisch aus DNA-Molekülen, die den Hauptteil des herzustellenden Expressionskonstruktes bilden, und bakteriellen Restsequenzen wie Antibiotikaresistenzgenen, Replikationsursprüngen u.v.m., der Ligation mit kurzen, haarnadelförmigen DNA-Oligomeren zugeführt. Besagte Oligomere tragen dabei einen einzelsträngigen Überhang, der auf den oder die einzelsträngigen Überhänge der Expressionskonstrukte passt und so zu einem hantelförmigen Expressionskonstrukt führt.

Kern der Erfindung ist nun, daß aus dem entstehenden Rohgemisch aus hantelförmigen Expressionskonstrukten sowie Restsequenzen, die durch die Behandlung mit Haarnadel-Oligomeren und Ligase ebenfalls zum größten Teil als hantelförmige Moleküle vorliegen, letztere durch zwei enzymatische Schritte vollständig entfernt werden: zuerst werden die unerwünschten Moleküle durch Verdau mit einem Restriktionsenzym zerschnitten, dessen Erkennungssequenz nur auf den unerwünschten Fremdmolekülen liegt, so daß die als Produkt gewünschten Expressionskonstrukte kovalent geschlossen bleiben. In einem zweiten Schritt werden dann die geschnittenen Moleküle mit einer Exonuklease verdaut, so daß nur die gewünschten Moleküle im Reaktionsgemisch als Polymere verbleiben. Dies führt dazu, daß im Reaktionsgemisch nur eine Spezies langkettiger DNA-Moleküle vorliegt, wodurch diese der Aufreinigung durch bekannte Methoden der lonenaustauschchromatographie zugänglich ist. Das besagte Verfahren kann durch festphasengebundene Enzyme durchgeführt werden.

Werden zum Verdau des Plasmids bakterielle Restriktionsendonukleasen der Klasse I eingesetzt, so entstehen durch den Verdau Überhänge palindromischer Sequenz. Dies bedeutet, daß diese Überhänge einen Strang eines Doppeistrang-Motivs mit diadischer Symmetrie darstellen; eine andere Form dieser Aussage ist, daß alle von der betreffenden Endonuklease hergestellten Fragmente mit allen anderen von der betreffenden Endonuklease hergestellten Fragmenten ligieren können. Das führt dazu, daß man zur Vermeidung von Ligationsreaktionen zwischen den langen Expressionskassetten einen großen Überschuß an kurzen Oligodesoxyribonukleotiden zusetzten muß. Es mußte in Versuchen zur Ligation in Abhängigkeit von der Länge der zu ligierenden Fragmente und deren Konzentration ein bis zu 200-facher Überschuß an Oligomeren in Bezug auf die Enden der zu ligierenden Restriktionsfragmente eingesetzt werden. Dies ist der

Fall, weil die Restriktionsfragmente intra- und intermolekular mit anderen Restriktionsfragmenten reagieren können. Der genannte Überschuß an Haarnadel-Oligomeren verschiebt das Gleichgewicht von der intra- oder intermolekularen Reaktion eines polymeren Restriktionsfragmentes mit einem anderen ganz auf die Seite der Reaktion mit den Oligomeren, führt aber zu einem sehr hohen Anteil der Oligo-Dimere im Reaktionsgemisch. Neben den dadurch entstehenden Kosten ist daran nachteilig, daß diese Oligomere als teilweise einzelsträngige DNA-Moleküle in einem so hohen Überschuß enzymatische Reaktionen unspezifisch hemmen können.

Ein weiterer Aspekt der hier vorgestellten Erfindung ist, daß die Menge der haarnadelförmigen Oligodesoxynukleotide, die zur Ligation an die durch Restriktionsverdau entstandenen Fragmente erheblich vermindert werden kann, wenn man die Ligation in Anwesenheit von Restriktionsendonukleasen ablaufen läßt, die im Verlaufe der Ligation entstehenden unerwünschten Verbindungen aus Ligationspartnern in-situ wieder lösen. Ligieren dabei zwei aus Verdau mit der gleichen Endonuklease entstandene polymere Restriktionsfragmente miteinander, so rekonstituieren sie wieder die ursprüngliche Schnittstelle. Diese Reaktion läßt sich in-situ durch Verdau mit der betreffenden Endonuklease rückgängig machen.

Um dabei die Ligation von Oligomeren an die Restriktionsfragmente nicht ebenfalls wieder rückgängig zu machen, muß die direkt neben dem Überhang liegende Sequenz der Oligomere so gewählt werden, daß sie bei Ligation an die Restriktionsfragmente die Schnittstelle nicht rekonstituiert. Vorteilhafterweise wird diese Sequenz vielmehr so gewählt, daß eine nicht palindromische Sequenz bei Ligation des Oligomers an den Überhang des Restriktionsfragmentes entsteht, die vor weiterem Verdau geschützt ist, und ebenfalls so, daß bei Reaktion des Oligomers mit einem weiteren Exemplar Oligomer passender Sequenz ein Dimer entsteht, so daß eine palindromische Sequenz entsteht, die durch eine zweite, in der Reaktionslösung enthaltene Endonuklease wieder gespalten werden kann. Auf diese Weise können in einem Eintopfverfahren durch eine Ligase und zwei Endonukleasen relativ kleine Überschüsse Haamadel-Oligomere (ca. 10- bis 30-fach) ausreichen, zu einem definierten Produkt aus hantelförmigen Monomeren des Expressionskonstruktes sowie der begleitenden, später abgetrennten Fremdsequenzen zu kommen. Dies kann zum Beispiel dadurch erreicht werden, daß man zum Verdau in das Plasmid eine die Expressionskassette flankierende Erkennungssequenz für die Endonuklease EcoRI (G'AATTC; "'" bezeichnet die Schnittstelle, Überhang AATT) einbaut, die 5'-überhängende Sequenz des Oligos mit AATT beginnen läßt und den sich daran anschließenden Doppelstrangbereich des Haarnadel-Oligos mit G beginnen läßt. Auf diese Weise entsteht bei Ligation zwischen dem Haamadel-Oligo und der Expressionskassette eine nicht-palindromische Sequenz GAATTG, bei Reaktion einer Expressionskassette mit einer anderen wird die EcoRI-Schnittstelle Wiederhergestellt, und bei Reaktion eines Oligos mit einem anderen kann das entstehende Dimer durch die Endonuklease Munl geschnitten werden (siehe Beispiel 1).

Ein weiterer Aspekt der Erfindung ist die Verwendung von sogenannten Klasse-II Endonukleasen, welche im Gegensatz zu den heute zumeist verwendeten Klasse-I Endonukleasen keine palindromischen Sequenzen erkennen (Butkus et al., Biochim Biophys Acta 1985 Dec 18;826(4):208-12 A new restriction endonuclease Eco31I recognizing a non-palindromic sequence). Durch die Entstehung nichtpalindromischer Überhänge können die Enden nicht mehr mit jedem anderen Ende reagieren. Da bei den Klasse-II-Endonukleasen die Schnittstellen neben den Erkennungssequenzen liegen, können auch die Reaktivitäten von Edukt und Produkt getrennt werden. Durch Platzierung der Erkennungssequenz auf dem Plasmid jenseits der gewünschten Produktsequenz kann ein religiertes Fragment des Plasmids durch in der Ligationslösung anwesende Endonuklease immer wieder geschnitten werden, während die mit Oligonukleotid ligierte Expressionskassette unreaktiv bleibt. Auf diese Weise kann die Menge an Oligodesoxyribonukleotid auf etwa einen fünffachen Überschuß in Enden gesenkt werden.

Bevorzugt wird zum Primärverdau eine Endonuklease der Gruppe der Klasse-II-Restriktionsendonukleasen, vorzugsweise ein Enzym aus der Gruppe BbsI, BbvI, BbvII, BpiI; BplI, BsaI, BsmAI, BsmBI, BsmFI, BspMI, Eam1104I, EarI, Eco31I, Esp3I, FokI, HgaI, SfaNI oder deren Isoschizomere eingesetzt.

Ein weiterer Aspekt der Erfindung ist, daß der Verdau der DNA-Präparation mittels sequenzspezifischer Endonukleasen sowie nachfolgendem Verdau mit sequenzunspezifischen Exönukleasen alle DNA aus der Präparation zu entfemen vermag, welche der Endonukiease eine Erkennungssequenz liefert und als Verunreinigung in der DNA enthalten ist. Heute in der medizinischen Anwendung bzw. klinischen Prüfung befindliche DNA-Plasmidpräparationen weisen einen erheblichen Grad an Verunreinigung mit genomischer DNA der zur Herstellung verwendeten Mikroorganismen auf. So spezifiziert ein führender Hersteller von GMP-zugelassener DNA für klinische Prüfungen den Gehalt an "Host DNA auf <5% (Quelle: Katalog der Qiagen GmbH auf dem Internet, Eintrag vom 11.06.1999 auf der Seite http://www.qiagen.com/catalog/chapter 12/chap12b.asp). Es ist anzunehmen, daß im Vergleich mit den Reinheitsstandards herkömmlicher Arzneimittel hier erheblicher Bedarf an Verbesserung besteht.

### Beispiel 1:

1 mg des Plasmids pG-EGFP (Sequenz siehe Anhang) wurde mit der Restriktionsendonuklease Eco RI vollständig verdaut. Die entstandenen Fragmente wurden mit 1 mg des 5'-phosphorylierten Desoxyoligoribonukleotids mit der Sequenz AATTGGCCGGCCGTTTCGGCCGGCC (TIB Molbiol, Berlin) in Anwesenheit von 100 U T4 DNA-Ligase (MBI-Fermentas, Vilnius, Litauen) sowie 50 u Restriktionsendonukleasen Munl und EcoRI in 5 ml Reaktionspuffer bei 37°C über Nacht umgesetzt. Die Reaktion wurde durch Erhitzen auf 60°C gestoppt.

Das entstandene Reaktionsgemisch wurde konzentriert und nach Umpuffern mit 100 U Restriktionsendonuklease HindIII sowie 100 U T7-DNA-Polymerase in Abwesenheit von Desoxyribonukleotiden 1 h verdaut. Das entstandene Produkt wurde per Anionen-Austausch-Chromatographie gereinigt und war nach Kontrolle durch Gelelektrophorese sowie PCR-Kontrolle frei von Resten des unerwünschten Fragmentes.

### Beispiel 2:

1 mg des Plasmids pMol-EGFP (Sequenz siehe Anhang) wurde mit der Restriktionsendonuklease Eco31I vollständig verdaut. Die entstandenen Fragmente wurden mit 50 µg des 5'-phosphorylierten Desoxyoligoribonukleotids mit der Sequenz AGGGGTCCAG TTTTCTGGAC (TIB Molbiol, Berlin) in Anwesenheit von 20 U T4 DNA-Ligase (MBI-Fermentas, Vilnius, Litauen) sowie 10 u Eco31I in 5 ml Reaktionspuffer bei 37°C über Nacht umgesetzt. Die Reaktion wurde durch Erhitzen auf 60°C gestoppt.

Das entstandene Reaktionsgemisch wurde konzentriert und nach Umpuffem mit 100 U Restriktionsendonuklease HindIII sowie 100 U T7-DNA-Polymerase in Abwesenheit von Desoxyribonukleotiden ü.N. verdaut. Das entstandene Produkt wurde per Anionen-Austausch-Chromatographie gereinigt und war nach Kontrolle durch Gelelektrophorese sowie PCR-Kontrolle frei von Resten des unerwünschten Fragmentes.

## Patentansprüche

1. Verfahren zur Herstellung eines hantelförmigen DNA-Moleküls, bestehend aus den folgenden Verfahrensschritten:
a) Vermehrung des das Molekül hauptsächlich bildenden DNA-Doppelstranges als Teil eines bakteriell replizierbaren Konstrukts,
b) isolierung des bakteriell replizierbaren Konstrukts,
c) Ausschneiden des das Molekül hauptsächlich bildenden DNA-Doppelstranges vermittels einer oder mehrerer DNA-Restriktionseridonukleasen zum Primärverdau, deren Schnittstellen die Sequenz des das Molekül hauptsächlich bildenden DNA-Doppelstranges an beiden Seiten unmittelbar einschließen, und deren Aktivität an der Schnittstelle kurze überstehende Enden aus einzelsträngiger DNA hinterlässt,
d) Ligation der so entstandenen Restriktionsfragmente mit Haarnadel-förmigen Oligodesoxyribonukleotiden, die kurze, auf die besagten Restriktionsfragmentenden passende Einzelstrangüberhänge aufweisen, so dass bei der Ligationsreaktion zwischen Oligodesoxyribonukleotid und Restriktionsfragment an beiden Seiten ein kovalent geschlossener Einzelstrang entsteht,
e) anschließendem Verdau des entstandenen Reaktionsgemisches vermittels Restriktionsendonuklease zum Sekundärverdau, die eine auf dem herzustellenden Molekül nicht vorhandene, auf dem Rest des bakteriell replizierbaren Konstrukts aber mindestens einmal vorhandene Erkennungssequenz erkennt und schneidet,
f) und anschließendem oder gleichzeitigem Verdau der Reaktionsmischung vermittels einer ausschließlich für freie 3'- oder 5'-DNA-Enden spezifischen Exonuklease.

2. Verfahren gemäß Anspruch 1, bei welchem das hantelförmige DNA-Molekül ein Expressionskonstrukt, bestehend aus Promoter, kodierender Sequenz und Terminatorsequenz, ist

3. Verfahren gemäß Anspruch 1, wobei die Haarnadel-förmigen Oligodesoxyribonukleotide eine solche Sequenz in ihrem Einzelstrangüberhang aufweisen, dass bei der Ligationsreaktion nach Verfahrensschritt d) zwischen Oligodesoxyribonuleotid und polymerem Restriktionsfragment keine palindromische Sequenz entsteht, und wobei während der Ligationsreaktion nach Verfahrensschritt d) solche Restriktionsendonukleasen anwesend sind, dass durch intraund intermolekulare Ligationsreaktionen der polymeren Restriktionsfragmente oder der Oligomere entstehende Erkennungssequenzen für Restriktionsendonukleasen diese in situ wieder geschnitten werden.

4. Verfahren gemäß Anspruch 1, bei welchem die besagte Endonuklease zum Primärverdau ein Enzym der Gruppe der Klasse-II-Restriktionsendonukleasen, vorzugsweise ein Enzym aus der Gruppe BbsI, BbvI, BbvII, BpiI; BplI, BsaI, BsmAI, BsmBI, BsmFI, BspMI, Eam1104I, EarI, Eco31I, Esp3I, FokI, HgaI, SfaNI oder deren Isoschizomere ist.

5. Verfahren nach Anspruch 1, 2 oder 3, bei dem das Verfahren durch festphasengebundene Enzyme durchgeführt wird.

6. Verfahren nach einem oder mehreren der Ansprüche 1 bis 5, wobei Präparationen hantelförmiger DNA-Moleküle gereinigt werden, indem die DNA-Präparation
a) einem Endonukleaseabbau der Ligationsprodukte zur Reinigung des gewünschten Ligationsproduktes
(b) einem Verdau der mit Exonukleasen, welche für freie Hydroxy- oder Phosphatgruppen von Desoxyribonukleinsäure spezifisch sind, vorzugsweise die Exonukleaseaktivitäten der DNA-Polymerase der Bakteriophagen T4 oder T7,
c) einer Abtrennung der verwendeten Enzyme, Pufferbestandteile sowie hydrolysierten Nukleotide,
unterzogen wird.

7. Verfahren nach Anspruch 6, wobei die Präparationen einem Verdau durch eine oder einer Vielzahl von Restriktionsendonukleasen in einem für die Aktivität der besagten Restriktionsendonukleasen geeigneten Medium ausgesetzt wird, wobei die zu reinigenden DNA-Moleküle keine Erkennungssequenzen für die verwendeten Restriktionsendonukleasen aufweisen.

8. Verfahren nach Anspruch 6 oder 7, wobei die DNA-Präparation anschließend oder gleichzeitig einem Verdau mit Exonukleasen, welche für freie Hydroxyoder Phosphatgruppen von Desoxyribonukleinsäure spezifisch sind, vorzugsweise die die Exonukleaseaktivitäten der DNA-Polymerase der Bakteriophagen T4 oder T7, ausgesetzt und dann einer Abtrennung der verwendeten Enzyme, Pufferbestandteile sowie hydrolysierten Nukleotide unterzogen wird.

## Claims

1. Method to obtain a dumbbell-shaped DNA molecule, consisting of the following steps:
a) multiplication of the DNA double strand comprising the major part of the molecule, as part of a construct that can replicate in bacteria,
b) isolation of the construct that can replicate in bacteria,
c) excision of the of the DNA double strand comprising the major part of the molecule, by means of one or several DNA restriction endonucleases for primary digestion, whereby the restriction sites of said endonucleases immediately flank the sequence of the molecule comprising the major part of the molecule on both sides, and the activity of said endonucleases leaves short, single-stranded protruding ends at the restriction site,
d) ligation of the restriction fragments thus formed, with hairpin-shaped oligodesoxyribonucleotides comprising short, single-stranded protruding ends that pair with said single-stranded protruding ends on said restriction fragments, so that a covalently closed single strand forms on both sides as a consequence of the ligation reaction between said oligodesoxynucleotides and said restriction fragments.
e) subsequent digestion of the reaction mixture thus obtained, with a restriction endonuclease for secondary digestion, which recognises and cuts a recognition site not present on the molecule that is to be obtained, but is present at least once on the remainder of the construct that can replicate in bacteria,
f) and subsequent or concurrent digestion of the reaction mixture with a exonuclease exclusively specific for free 3' or 5' ends of DNA.

2. Method according to claim 1, where the dumbbell-shaped DNA molecule is an expression construct comprising a promoter sequence, a coding sequence and a terminator sequence.

3. Method according to claim 1, where the hairpin-shaped oligodesoxyribonucleotides comprise such a sequence in their single-stranded protruding ends, that upon the ligation reaction according to step d) between oligodeoxyribonucleotide and polymeric restriction fragment, no palindromic sequence forms, and where restriction endonucleases are present in the ligation reaction of step d) so that restriction sites formed as a consequence of intra-molecular or intermolecular ligation reactions of the restriction fragments or the oligomers are being digested in-situ.

4. Method according to claim 1, where said endonuclease for primary digestion is an enzyme being a member of the group of class II endonucleases, preferably an enzyme of the group BbsI, BbvI, BbvII, BpiI; BplI, BsaI, BsmAI, BsmBI, BsmFI, BspMI, Eam1104I, EarI, Eco31I, Esp3I, FokI, HgaI, SfaNI or an isoschizomer thereof.

5. Method according to claim 1, 2 or 3, where the method is being performed by means of enzymes bound to a solid phase.

6. Method according to one or several of the claims 1 to 5, where preparations of dumbbell-shaped molecules are purified by submitting the DNA-preparation to
a) an endonuclease digestion of the ligation products to purify the desired ligation product,
b) a digestion with exonucleases specific for free hydroxy- or phosphate groups of DNA, preferably the exonuclease activity of the DNA polymerase derived from bacteriophages T4 or T7, and
c) a separation of the hydrolyzed nucleotides, enzymes and buffer components employed in the reaction.

7. Method according to claim 6, where the preparations are submitted to a digestion of one or several restriction endonucleases in a buffer suited to the activity of said endonucleases, where the DNA molecules that are to be purified do not comprise a recognition sequence for said restriction endonucleases employed in the reaction.

8. Method according to claim 6 or 7, where the DNA preparation is submitted, subsequently or concurrently, to a digestion by exonucleases that are specific for free hydroxy- or phosphate groups of DNA, preferably the exonuclease activity of the DNA polymerase derived from bacteriophages T4 or T7, and where the DNA preparation is subsequently submitted to a separation of the hydrolyzed nucleotides, enzymes and buffer components employed in the reaction.

## Revendications

1. Procédé de production d'une molécule d'ADN en forme d'haltère, comprenant les étapes de procédé suivantes :
a) multiplication du double brin d'ADN formant essentiellement la molécule en tant que partie d'un construct bactérien réplicable,
b) isolation du construct bactérien réplicable,
c) dissociation ou découpage du double brin d'ADN formant essentiellement la molécule au moyen d'une ou de plusieurs endonucléases de restriction d'ADN à des fins de digestion primaire, dont les points de coupure incorporent directement au niveau des deux côtés la séquence du double brin d'ADN formant essentiellement la molécule, et dont l'activité laisse au niveau du point de coupure de courtes extrémités saillantes d'ADN simple brin,
d) ligature des fragments de restriction ainsi obtenus avec des oligodésoxyribonucléotides en forme d'épingle à cheveu qui présentent des prolongements de simple brin adaptés aux dits fragments de restriction, de sorte que lors de la réaction de ligature se forme entre l'oligodésoxyribonucléotide et le fragment de restriction, des deux côtés, un simple brin fermé de façon covalente,
e) digestion consécutive du mélange réactionnel obtenu au moyen d'une endonucléase de restriction à des fins de digestion secondaire, qui reconnaît et coupe une séquence de reconnaissance non présente sur la molécule à préparer mais existant au moins une fois sur le reste du construct bactérien réplicable,
f) et digestion consécutive ou simultanée du mélange réactionnel au moyen d'une exonucléase exclusivement spécifique des extrémités d'ADN 3' ou 5'.

2. Procédé selon la revendication 1, dans lequel la molécule d'ADN en forme d'haltère est un construct d'expression composé d'un promoteur, d'une séquence codante et d'une séquence terminateur.

3. Procédé selon la revendication 1, dans lequel l'oligodésoxyribonucléotide en forme d'épingle à cheveu présente une séquence dans son prolongement de simple brin telle que lors de la réaction de ligature selon l'étape d), il ne se forme pas de séquence palindromique entre l'oligodésoxyribonucléotide et le fragment de restriction polymère, et dans lequel, pendant la réaction de ligature selon l'étape d) sont présentes de telles endonucléases de restriction de sorte que, par des réactions de ligatures intra- et intermoléculaires des fragments polymères de restriction ou des oligomères, des séquences de reconnaissance des endonucléases de restriction se formant coupent à nouveau celles-ci in situ.

4. Procédé selon la revendication 1, dans lequel ladite endonucléase de digestion primaire est une enzyme du groupe des endonucléases de restriction de type II, de préférence une enzyme du groupe de BbsI, BbvI, BbvII, Bpil, BplI, BsaI, BsmAI, BsmBI, BsmFI, BspMI, Eam1104I, EarI, Eco31I, Esp3I, Fokl, HgaI, SfaNI ou leurs isoschizomèrcs.

5. Procédé selon la revendication 1, 2 ou 3, dans lequel le procédé est conduit par des enzymes liées en phase solide.

6. Procédé selon une ou plusieurs des revendications 1 à 5, dans lequel des préparations de molécules d'ADN en forme d'haltères sont purifiées en ce que la préparation d'ADN est soumise à
a) une dégradation par endonucléase des produits de ligature afin de purifier le produit de ligature souhaité,
b) une digestion avec des exonucléases qui sont spécifiques des groupes hydroxy ou phosphate libres des acides désoxyribonucléiques, de préférence présentant des activités d'exonucléases de l'ADN-polymérase des bactériophages T4 ou T7,
c) une séparation des enzymes utilisées, des composants du tampon et des nucléotides hydrolysés.

7. Procédé selon la revendication 6, dans lequel les préparations sont soumises à une digestion par une ou plusieurs endonucléases de restriction dans un milieu approprié pour l'activité desdites endonucléases de restriction, où les molécules d'ADN à purifier ne présentent pas de séquence de reconnaissance des endonucléases de restriction utilisées.

8. Procédé selon la revendication 6 ou 7, dans lequel la préparation d'ADN est soumise ensuite ou simultanément à une digestion avec des exonucléases qui sont spécifiques des groupes hydroxy ou phosphate libres des acides désoxyribonucléiques, de préférence soumises aux activités d'exonucléases de l'ADN-polymérase des bactériophages T4 ou T7, et ensuite soumise à une séparation des enzymes utilisées, des composants du tampon et des nucléotides hydrolysés.


### SEQUENZPROTOKOLL

<110> Mologen GmbH
<120> Verfahren zur Herstellung von hantelförmigen DNA-Molekülen als Expressionskonstrukte
<130> EP 0 967 274
<140> EP 99250187.4
   <141> 1999-06-14
<150> DE 198 26 758
   <151> 1998-06-15
<160> 2
<170> PatentIn Ver. 2.1
<210> 1
   <211> 4397
   <212> DNA
   <213> Künstliche Sequenz
<220>
   <223> Beschreibung der künstlichen Sequenz: Sequenz 1: Plasmid (pG-EGFP)
<400> 1
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttgcga attctggatc cgctagctta accgtattac cgccatgcat tagttattaa 2460
   tagtaatcaa ttacggggtc attagttcat agcccatata tggagttccg cgttacataa 2520
   cttacggtaa atggcccgcc tggctgaccg cccaacgacc cccgcccatt gacgtcaata 2580
   atgacgtatg ttcccatagt aacgccaata gggactttcc attgacgtca atgggtggag 2640
   tatttacggt aaactgccca cttggcagta catcaagtgt atcatatgcc aagtacgccc 2700
   cctattgacg tcaatgacgg taaatggccc gcctggcatt atgcccagta catgacctta 2760
   tgggactttc ctacttggca gtacatctac gtattagtca tcgctattac catggtgatg 2820
   cggttttggc agtacatcaa tgggcgtgga tagcggtttg actcacgggg atttccaagt 2880
   ctccacccca ttgacgtcaa tgggagtttg ttttggcacc aaaatcaacg ggactttcca 2940
   aaatgtcgta acaactccgc cccattgacg caaatgggcg gtaggcgtgt acggtgggag 3000
   gtctatataa gcagagctgg tttagtgaac cgtcagatgg taccgaagcc gctagcgcta 3060
   ccggtcgcca ccatggtgag caagggcgag gagctgttca ccggggtggt gcccatcctg 3120
   gtcgagctgg acggcgacgt aaacggccac aagttcagcg tgtccggcga gggcgagggc 3180
   gatgccacct acggcaagct gaccctgaag ttcatctgca ccaccggcaa gctgcccgtg 3240
   ccctggccca ccctcgtgac caccctgacc tacggcgtgc agtgcttcag ccgctacccc 3300
   gaccacatga agcagcacga cttcttcaag tccgccatgc ccgaaggcta cgtccaggag 3360
   cgcaccatct tcttcaagga cgacggcaac tacaagaccc gcgccgaggt gaagttcgag 3420
   ggcgacaccc tggtgaaccg catcgagctg aagggcatcg acttcaagga ggacggcaac 3480
   atcctggggc acaagctgga gtacaactac aacagccaca acgtctatat catggccgac 3540
   aagcagaaga acggcatcaa ggtgaacttc aagatccgcc acaacatcga ggacggcagc 3600
   gtgcagctcg ccgaccacta ccagcagaac acccccatcg gcgacggccc cgtgctgctg 3660
   cccgacaacc actacctgag cacccagtcc gccctgagca aagaccccaa cgagaagcgc 3720
   gatcacatgg tcctgctgga gttcgtgacc gccgccggga tcactctcgg catggacgag 3780
   ctgtacaaga gctcataatc agccatacca catttgtaga ggttttactt gctttaaaaa 3840
   acctcccaca cctccccctg aacctgaaac ataaaatgaa tgcaattctt gttgttaact 3900
   tgtttattgc agcttataat ggttacaaat aaagcaatag catcacaaat ttcacaaata 3960
   aagcattttt ttcactgcat tctagttgtg gtttgtccaa actcatcaat gtatcttaac 4020
   gcgaattgtt gttgttaact tgtttattgc agcttataat ggttacaaat aaagcaatag 4080
   catcacaaat ttcacaaata aagcattttt ttcactgcat tctagttgtg gtttgtccaa 4140
   actcatcaat gtatcttaac gcgaattcgt aatcatggtc atagctgttt cctgtgtgaa 4200
   attgttatcc gctcacaatt ccacacaaca tacgagccgg aagcataaag tgtaaagcct 4260
   ggggtgccta atgagtgagc taactcacat taattgcgtt gcgctcactg cccgctttcc 4320
   agtcgggaaa cctgtcgtgc cagctgcatt aatgaatcgg ccaacgcgcg gggagaggcg 4380
   gtttgcgtat tgggcgc                                                4397
<210> 2
   <211> 4278
   <212> DNA
   <213> Künstliche Sequenz
<220>
   <223> Beschreibung der künstlichen Sequenz:Sequenz 2: Plasmid (pMOL-EGFP)
<400> 2
   tcttccgctt cctcgctcac tgactcgctg cgctcggtcg ttcggctgcg gcgagcggta 60
   tcagctcact caaaggcggt aatacggtta tccacagaat caggggataa cgcaggaaag 120
   aacatgtgag caaaaggcca gcaaaaggcc aggaaccgta aaaaggccgc gttgctggcg 180
   tttttccata ggctccgccc ccctgacgag catcacaaaa atcgacgctc aagtcagagg 240
   tggcgaaacc cgacaggact ataaagatac caggcgtttc cccctggaag ctccctcgtg 300
   cgctctcctg ttccgaccct gccgcttacc ggatacctgt ccgcctttct cccttcggga 360
   agcgtggcgc tttctcatag ctcacgctgt aggtatctca gttcggtgta ggtcgttcgc 420
   tccaagctgg gctgtgtgca cgaacccccc gttcagcccg accgctgcgc cttatccggt 480
   aactatcgtc ttgagtccaa cccggtaaga cacgacttat cgccactggc agcagccact 540
   ggtaacagga ttagcagagc gaggtatgta ggcggtgcta cagagttctt gaagtggtgg 600
   cctaactacg gctacactag aaggacagta tttggtatct gcgctctgct gaagccagtt 660
   accttcggaa aaagagttgg tagctcttga tccggcaaac aaaccaccgc tggtagcggt 720
   ggtttttttg tttgcaagca gcagattacg cgcagaaaaa aaggatctca agaagatcct 780
   ttgatctttt ctacggggtc tgacgctcag tggaacgaaa actcacgtta agggattttg 840
   gtcatgagat tatcaaaaag gatcttcacc tagatccttt taaattaaaa atgaagtttt 900
   aaatcaatct aaagtatata tgagtaaact tggtctgaca gttaccaatg cttaatcagt 960
   gaggcaccta tctcagcgat ctgtctattt cgttcatcca tagttgcctg actccccgtc 1020
   gtgtagataa ctacgatacg ggagggctta ccatctggcc ccagtgctgc aatgataccg 1080
   cgagacccac gctcaccggc tccagattta tcagcaataa accagccagc cggaagggcc 1140
   gagcgcagaa gtggtcctgc aactttatcc gcctccatcc agtctattaa ttgttgccgg 1200
   gaagctagag taagtagttc gccagttaat agtttgcgca acgttgttgc cattgctaca 1260
   ggcatcgtgg tgtcacgctc gtcgtttggt atggcttcat tcagctccgg ttcccaacga 1320
   tcaaggcgag ttacatgatc ccccatgttg tgcaaaaaag cggttagctc cttcggtcct 1380
   ccgatcgttg tcagaagtaa gttggccgca gtgttatcac tcatggttat ggcagcactg 1440
   cataattctc ttactgtcat gccatccgta agatgctttt ctgtgactgg tgagtactca 1500
   accaagtcat tctgagaata gtgtatgcgg cgaccgagtt gctcttgccc ggcgtcaata 1560
   cgggataata ccgcgccaca tagcagaact ttaaaagtgc tcatcattgg aaaacgttct 1620
   tcggggcgaa aactctcaag gatcttaccg ctgttgagat ccagttcgat gtaacccact 1680
   cgtgcaccca actgatcttc agcatctttt actttcacca gcgtttctgg gtgagcaaaa 1740
   acaggaaggc aaaatgccgc aaaaaaggga ataagggcga cacggaaatg ttgaatactc 1800
   atactcttcc tttttcaata ttattgaagc atttatcagg gttattgtct catgagcgga 1860
   tacatatttg aatgtattta gaaaaataaa caaatagggg ttccgcgcac atttccccga 1920
   aaagtgccac ctgacgtcta agaaaccatt attatcatga cattaaccta taaaaatagg 1980
   cgtatcacga ggccctttcg tctcgcgcgt ttcggtgatg acggtgaaaa cctctgacac 2040
   atgcagctcc cggagacggt cacagcttgt ctgtaagcgg atgccgggag cagacaagcc 2100
   cgtcagggcg cgtcagcggg tgttggcggg tgtcggggct ggcttaacta tgcggcatca 2160
   gagcagattg tactgagagt gcaccatatg cggtgtgaaa taccgcacag atgcgtaagg 2220
   agaaaatacc gcatcaggcg ccattcgcca ttcaggctgc gcaactgttg ggaagggcga 2280
   tcggtgcggg cctcttcgct attacgccag ctggcgaaag ggggatgtgc tgcaaggcga 2340
   ttaagttggg taacgccagg gttttcccag tcacgacgtt gtaaaacgac ggccagtgcc 2400
   aagcttggtc tccccctgga tccgctagct taaccgtatt accgccatgc attagttatt 2460
   aatagtaatc aattacgggg tcattagttc atagcccata tatggagttc cgcgttacat 2520
   aacttacggt aaatggcccg cctggctgac cgcccaacga cccccgccca ttgacgtcaa 2580
   taatgacgta tgttcccata gtaacgccaa tagggacttt ccattgacgt caatgggtgg 2640
   agtatttacg gtaaactgcc cacttggcag tacatcaagt gtatcatatg ccaagtacgc 2700
   cccctattga cgtcaatgac ggtaaatggc ccgcctggca ttatgcccag tacatgacct 2760
   tatgggactt tcctacttgg cagtacatct acgtattagt catcgctatt accatggtga 2820
   tgcggttttg gcagtacatc aatgggcgtg gatagcggtt tgactcacgg ggatttccaa 2880
   gtctccaccc cattgacgtc aatgggagtt tgttttggca ccaaaatcaa cgggactttc 2940
   caaaatgtcg taacaactcc gccccattga cgcaaatggg cggtaggcgt gtacggtggg 3000
   aggtctatat aagcagagct ggtttagtga accgtcagat ggtaccgaag ccgctagcgc 3060
   taccggtcgc caccatggtg agcaagggcg aggagctgtt caccggggtg gtgcccatcc 3120
   tggtcgagct ggacggcgac gtaaacggcc acaagttcag cgtgtccggc gagggcgagg 3180
   gcgatgccac ctacggcaag ctgaccctga agttcatctg caccaccggc aagctgcccg 3240
   tgccctggcc caccctcgtg accaccctga cctacggcgt gcagtgcttc agccgctacc 3300
   ccgaccacat gaagcagcac gacttcttca agtccgccat gcccgaaggc tacgtccagg 3360
   agcgcaccat cttcttcaag gacgacggca actacaagac ccgcgccgag gtgaagttcg 3420
   agggcgacac cctggtgaac cgcatcgagc tgaagggcat cgacttcaag gaggacggca 3480
   acatcctggg gcacaagctg gagtacaact acaacagcca caacgtctat atcatggccg 3540
   acaagcagaa gaacggcatc aaggtgaact tcaagatccg ccacaacatc gaggacggca 3600
   gcgtgcagct cgccgaccac taccagcaga acacccccat cggcgacggc cccgtgctgc 3660
   tgcccgacaa ccactacctg agcacccagt ccgccctgag caaagacccc aacgagaagc 3720
   gcgatcacat ggtcctgctg gagttcgtga ccgccgccgg gatcactctc ggcatggacg 3780
   agctgtacaa gtgagctcat aatcagccat accacatttg tagaggtttt acttgcttta 3840
   aaaaacctcc cacacctccc cctgaacctg aaacataaaa tgaatgcaat tgttgttgtt 3900
   aacttgttta ttgcagctta taatggttac aaataaagca atagcatcac aaatttcaca 3960
   aataaagcat ttttttcact gcattctagt tgtggtttgt ccaaactcat caatgtatct 4020
   taacgcgaat tcagggggag acccaattcg taatcatggt catagctgtt tcctgtgtga 4080
   aattgttatc cgctcacaat tccacacaac atacgagccg gaagcataaa gtgtaaagcc 4140
   tggggtgcct aatgagtgag ctaactcaca ttaattgcgt tgcgctcact gcccgctttc 4200
   cagtcgggaa acctgtcgtg ccagctgcat taatgaatcg gccaacgcgc ggggagaggc 4260
   ggtttgcgta ttgggcgc 4278